# EUROPEAN PATENT APPLICATION

(11) **EP 2 638 898 A1**
(43) Date of publication of application: **18.09.2013**
(21) Application number: 13159426.9
(22) Date of filing: 15.03.2013
(51) Int. Cl.: A61K 9/20, A61K 9/28, A61K 31/155, A61K 31/33, A61K 31/4439

(54) **Metformin and Pioglitazone Formulation with Different Release Profiles**

(30) Priority: 16.03.2012 TR 201203021
(71) Applicant: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(57) **Abstract**

The present invention relates to a bilayer tablet formulation providing a concomitant immediate release and controlled release.

## Description

### Field of Invention

The present invention relates to formulations made with metformin or a pharmaceutically acceptable salt of metformin and pioglitazone or a pharmaceutically acceptable salt of pioglitazone. The present invention more particularly relates to a formulation of metformin and pioglitazone, providing a concomitant immediate release and controlled release.

### Background of Invention

Metformin hydrochloride, with the chemical name 1,1-dimethylbiguanide hydrochloride, has the following chemical structure of Formula I.

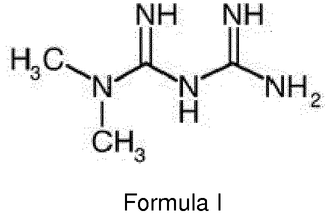

Metformin is an oral antidiabetics having an orally-administrated biguanide structure. It is used singly or in combination with sulfonylureas, alpha-glucosidase inhibitors, or insulin. It is freely dissolved in water.

Various patent applications are available in relation to metformin.

Metformin is disclosed with the patent US 3,174,901.

The patent US 6,475,521 discloses a water-soluble pharmaceutical formulation comprising antidiabetics metformin, providing prolonged release.

On the other hand, the chemical designation of pioglitazone hydrochloride is (+/-)-5-[[4-[2-(5-ethyl-2-pyridinyl)ethoxy]phenyl]methyl]-2,4-thiazolidinedione hydrochloride, with the following chemical structure of Formula 2.

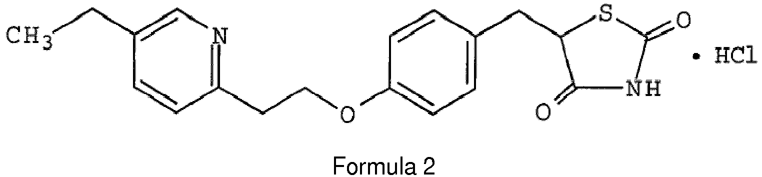

Pioglitazone is an orally-administered antidiabetics. It is the member of a group of medications, which are also called as thiazolidinediones and insulin sensitizers. It is not practically soluble in water.

Pioglitazone is disclosed in the patent EP0193256.

Various combination formulations have been disclosed, which comprise metformin and pioglitazone.

The patent EP0514452B2 discloses the use of an active agent selected among pioglitazone hydrochloride, metformin, indole amine antidiabetics, thermogenic β-agonists, CS 045, and thiazolidinedione derivatives, in preparing a medicament for use in treating or preventing hypertension in an insulin-resistant patient.

The application WO03105809 discloses a multilayer tablet composed of thiazolidinedione and biguanide. The tablet comprises immediate-release and controlled-release layers.

Tablets are known from the prior art, which comprise layers providing immediate release and controlled release. Immediate-release layers provide an immediate balance of the blood glucose amount, whereas controlled-release layers provide for a prolonged maintenance of the blood sugar proportion. However, an undesired case is encountered in these embodiments. Disintegration and dissolution take place in the controlled-release layer and immediate-release layer following drug administration such that the active agent is diffused into the present medium. If, however, the immediate-release layer does not disintegrate rapidly enough, the polymers in the controlled-release layers providing and controlling the release form a diffusion barrier, which completely surrounds the tablet. This barrier prevents the disintegration of the immediate-release layer which does not disintegrate rapidly enough and avoids the active agent from exhibiting its desired activity. Thus, the desired release profile cannot be achieved. Additional time is required for the commencement of activity of currently-available formulations. This is not desirable for the patient. A quick onset of the treatment process directly affects the life quality of patients.

Furthermore, the solubility of the metformin molecule is high, whereas the solubility of the pioglitazone molecule is low. It is quite difficult to achieve the targeted release profile.

The selection and proportion of the excipients, which are to provide the desired release profile, and at the same time provide a desired rate for the immediate-release layer are quite critical.

Considering the aforesaid problems, it is obvious that a novelty is needed in both providing production convenience in the art of "once a day" dose formulations containing metformin hydrochloride and pioglitazone, and ensuring convenient release profiles for the same.

### Object and Brief Description of Invention

The present invention provides a combination formulation of metformin and pioglitazone, eliminating all aforesaid problems and bringing additional advantages to the relevant prior art.

Accordingly, the main object of the present invention is to obtain a formulation of metformin and pioglitazone, which is orally administered once or twice a day.

Another object of the present invention is to reduce the treatment onset time with said formulation providing a 24-hour effect.

A further object of the present invention is to provide a disintegration rate of the immediate-release layer, which is at a desired level.

A solid oral pharmaceutical formulation comprising a controlled-release layer containing metformin or a pharmaceutically acceptable salt or polymorph of metformin, and an immediate-release layer containing pioglitazone or a pharmaceutically acceptable salt or polymorph of pioglitazone has been developed to carry out all objects, referred to above and to emerge from the following detailed description.

In a preferred embodiment according to the present invention, said novelty is carried out in that the controlled-release layer comprises hydroxypropyl methylcellulose and the immediate-release layer comprises calcium carboxymethyl cellulose, and in that the proportion range of hydroxypropyl methylcellulose to calcium carboxymethyl cellulose is 0.7 to 40.

A preferred embodiment of the present invention also contains one or more excipient(s).

In an embodiment of the present invention, the proportion range of hydroxypropyl methylcellulose to calcium carboxymethyl cellulose is 0.7 to 25.

In a preferred embodiment of the present invention, the proportion range of hydroxypropyl methylcellulose to calcium carboxymethyl cellulose is 0.7 to 10.

In a preferred embodiment of the present invention, said excipients contain at least one or a mixture of binders, diluents, disintegrants, glidants, and lubricants.

In a preferred embodiment of the present invention, said binder is at least one or a mixture of polyvinylpyrrolidone (povidone), hydroxypropyl methylcellulose, hydroxypropyl cellulose, carboxymethyl cellulose, methyl cellulose, ethyl cellulose and similar cellulose derivatives and gelatin.

In another preferred embodiment of to the present invention, said diluents include at least one or a mixture of lactose, starch, mannitol, calcium hydrogen phosphate dihydrate, dicalcium hydrogen phosphate anhydrate, calcium phosphate trihydrate, silicium dioxide and glucose.

In a preferred embodiment of to the present invention, said glidants comprise at least one or a mixture of colloidal silicon dioxide, talk, aluminum silicate, and magnesium silicate.

In a preferred embodiment of the present invention, said formulation comprises magnesium stearate as a lubricant.

Another preferred embodiment of the present invention comprises the following ingredients only:
I. immediate-release layer
   a. 1.2-40% by weight of pioglitazone hydrochloride
   b. 1-25% by weight of hydroxypropyl cellulose
   c. 10-82% by weight of lactose monohydrate
   d. 1-20% by weight of calcium carboxymethyl cellulose
   e. 0.1-2% by weight of magnesium stearate
II. controlled-release layer
   a. 35-80% by weight of metformin hydrochloride
   b. 0.5-5% by weight of hydroxypropyl methyl cellulose (low viscosity)
   c. 15-40% by weight of hydroxypropyl methyl cellulose (high viscosity)
   d. 1.5-7.5% by weight of calcium carboxymethyl cellulose
   e. 0.1-2% by weight of magnesium stearate

A further preferred embodiment of the present invention provides a method for preparing a pharmaceutical formulation, this method comprising the steps of
a. mixing together metformin hydrochloride, carboxymethylcellulose sodium, and low viscosity hydroxypropyl methyl cellulose,
b. granulating the mixture with a sufficient amount of water,
c. sieving the wet granules formed, drying the same, and sieving the dried granules again,
d. adding high viscosity hydroxypropyl methyl cellulose into the resulting mixture and mixing the same,
e. adding magnesium stearate into the final mixture and mixing the same to give a controlled-release phase,
f. mixing together pioglitazone hydrochloride, lactose monohydrate, and calcium carboxymethyl cellulose,
g. adding an aqueous solution of hydroxypropyl cellulose prepared separately into this mixture to provide granules and drying the latter,
h. sieving magnesium stearate and mixing it into the resulting mixture to give the immediate-release phase,
i. compressing the controlled-release phase and the immediate-release phase to give a bilayer tablet.

### Detailed Description of Invention

### Brief Description of Figures

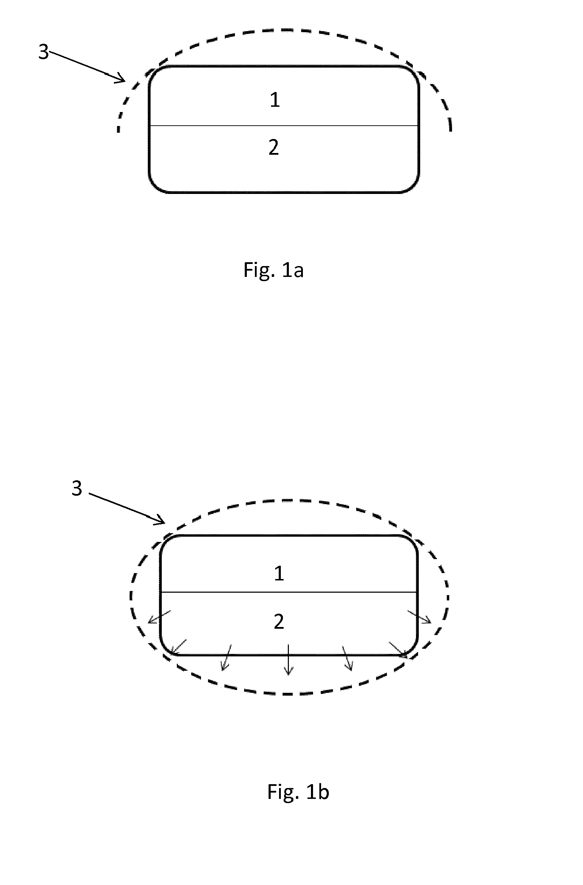

Figure 1a is a representative illustration of a bilayer tablet embodiment according to the present invention used in the state of art.
Figure 1b is a representative illustration of a bilayer tablet embodiment according to the present invention used in the state of art.

### Reference Numbers in Figures

1. controlled-release layer
2. immediate-release layer
3. diffusion barrier

In the following detailed description, the problem encountered in bilayer tablets according to the present invention in the state of art shall be described illustratively by making references to annexed figures only to make it clear without imposing any restrictions thereon.

As illustrated in figures 1 and 2, the bilayer tablet according to the present invention starts dissolving in the controlled-release layer (1) and in the immediate-release layer (3) following administration. If the immediate-release layer cannot dissolve at a desired rate, its activity is prevented by a diffusion barrier (3) formed by the controlled-release layer.

### Example

| **Metformin MR + Pioglitazone Tablet Formulation** | |
|---|---|
| **Immediate-release layer** | **% (mg/mg)** |
| Pioglitazone hydrochloride | 25 - 30 |
| Hydroxypropyl cellulose | 1 - 5 |
| Lactose monohydrate | 60 - 70 |
| Calcium carboxymethyl cellulose | 5 - 10 |
| Magnesium stearate | 0.1 - 1 |

| **Controlled-release layer** | |
|---|---|
| Metformin hydrochloride | 70 - 80 |
| Hydroxypropyl methyl cellulose | 20 - 25 |
| Sodium carboxymethylcellulose | 1 - 5 |
| Magnesium stearate | 0.1 - 1 |

### Preparing the immediate-release layer:

The immediate-release layer of the formulation is obtained as following.

Weighed amounts of pioglitazone hydrochloride, lactose monohydrate, and calcium carboxymethyl cellulose are charged to a fluidized bed dryer and mixed. An aqueous solution of an weighed amount of hydroxypropyl cellulose is prepared and is sprayed over the mixture present in the fluidized bed dryer and is thus granulated and dried. The prepared wet granules are sieved. Magnesium stearate is added to the sieved dry granules and is mixed approximately 3 minutes to give the immediate-release layer.

### Preparing the controlled-release layer:

The controlled-release layer of the formulation is obtained as following.

A weighed amount of metformin HCl is introduced into a granulator and mixed for 10 minutes. Then, carboxymethylcellulose sodium and low-viscosity hydroxypropyl methylcellulose (K100 LV) are added and mixed for 5 minutes. Water is sprayed onto the resulting mixture and is thus granulated. The granules are sieved and are added to the fluidized bed dryer and dried. The dried granules are passed through a sieve and ground.

High-viscosity hydroxypropyl methylcellulose (K100 MCR) is added to the sieved dry granules and mixed for around 15 minutes. Magnesium stearate is added into this mixture and is mixed for around 3 more minutes to give the controlled-release layer.

This invention has surprisingly provided a bilayer tablet formulation comprising metformin hydrochloride and pioglitazone hydrochloride, with a desired release profile. The proportions of the immediate-release and the controlled-release phases in said bilayer tablet are such determined that a formulation is obtained of convenient and desired quality, which provides drug release up to 24 hours. The controlled-release layer comprises hydroxypropyl methylcellulose, whereas the immediate-release layer comprises calcium carboxymethyl cellulose. The proportion range of hydroxypropyl methylcellulose to calcium carboxymethyl cellulose, which is 0.7 to 40, preferably 0.7 to 25, and more preferably 0.7 to 10, allows to obtain a desired release profile in the formulation which comprises highly water-soluble metformin and weakly water-soluble pioglitazone. The treatment onset can be realized at a desired level depending particularly on the dissolution rate of the immediate-release layer.

This formulation developed is used in the prevention or treatment or diabetes mellitus.

Although the obtained tablet is bilayered, it is alternatively possible to design said tablet with more layers as well. It is possible to provide one or more intermediate layer(s) between the layers containing the active agents in order to combine the layers, such intermediate layer(s) possibly including an active agent or not including an active agent, and providing immediate release or controlled release. Whilst both layers including active agents may provide controlled release or immediate release, at least one thereof may provide immediate release while the others provide controlled, prolonged, and retarded release.

It is also possible to use the following additional excipients in this formulation.

Binders, e.g. at least one or a mixture of polyvinylpyrrolidone, gelatin, sugars, glucose, natural gums, gums, synthetic celluloses, polymethacrylate, hydroxypropyl methylcellulose, hydroxypropyl cellulose, carboxymethyl cellulose, methyl cellulose, and other cellulose derivatives, etc..

Glidants, e.g. at least one or a mixture of colloidal silicon dioxide, talk, and aluminum silicate, etc..

Lubricants, e.g. at least one or a mixture of sodium stearyl fumarate, magnesium stearate, polyethylene glycol, stearic acid, metal stearates, boric acid, sodium chloride benzoate and acetate, sodium or magnesium lauryl sulfate, etc..

Disintegrants, e.g. at least one or a mixture of sodium starch glycolate, croscarmellose sodium, crospovidone, sodium alginate, gums, starch, and magnesium aluminum silicate, etc..

Surface active agents, e.g. at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, and magnesium lauryl sulfate, etc..

Coating agents, e.g. hydroxypropyl methylcellulose, polyethylene glycol, polyvinylpyrrolidone, polyvinylpyrrolidone-vinyl acetate copolymer (PVP-VA), polyvinyl alcohol and other polymers, and all kinds of OpadryTM, as well as pigments, dyes, titanium dioxide and iron oxide, talk, etc..

## Claims

1. A solid oral pharmaceutical formulation comprising a controlled-release layer containing metformin or a pharmaceutically acceptable salt or polymorph of metformin, and an immediate-release layer containing pioglitazone or a pharmaceutically acceptable salt or polymorph of pioglitazone, **characterized in that**
- said controlled-release layer comprises hydroxypropyl methylcellulose, whereas the immediate-release layer comprises calcium carboxymethyl cellulose, and
- the proportion range of hydroxypropyl methylcellulose to calcium carboxymethyl cellulose is 0.7 to 40.

2. The pharmaceutical formulation according to Claim 1, further comprising at least one or more excipients.

3. The pharmaceutical formulation according to any of the preceding claims, wherein the proportion range of hydroxypropyl methylcellulose to calcium carboxymethyl cellulose is preferably 0.7 to 25.

4. The pharmaceutical formulation according to any of the preceding claims, wherein the proportion range of hydroxypropyl methylcellulose to calcium carboxymethyl cellulose is more preferably 0.7 to 10.

5. The pharmaceutical formulation according to any of the preceding claims, wherein said excipient comprises at least one or a mixture of binders, diluents, disintegrants, glidants, and lubricants.

6. The pharmaceutical formulation according to any of the preceding claims, wherein said binders comprise at least one or a mixture of polyvinylpyrrolidone (povidone), hydroxypropyl methylcellulose, hydroxypropyl cellulose, carboxymethyl cellulose, methyl cellulose, ethyl cellulose and other cellulose derivatives, and gelatin.

7. The pharmaceutical combination according to any of the preceding claims, **characterized in that** said diluents comprise at least one or a mixture of lactose, starch, mannitol, calcium hydrogen phosphate dihydrate, dicalcium hydrogen phosphate anhydrate, calcium phosphate trihydrate, silicium dioxide, and glucose.

8. The pharmaceutical formulation according to any of the preceding claims, **characterized in that** said glidants comprise at least one or a mixture of colloidal silicon dioxide, talk, aluminum silicate, and magnesium silicate.

9. The pharmaceutical formulation according to any of the preceding claims, comprising magnesium stearate as the lubricant.

10. The pharmaceutical formulation according to any of the preceding claims, consisting of the following ingredients only:
I. immediate-release layer
a. 1.2-40% by weight of pioglitazone hydrochloride
b. 1-25% by weight of hydroxypropyl cellulose
c. 10-82% by weight of lactose monohydrate
d. 1-20% by weight of calcium carboxymethyl cellulose
e. 0.1-2% by weight of magnesium stearate
II. controlled-release layer
a. 35-80% by weight of metformin hydrochloride
b. 0.5-5% by weight of hydroxypropyl methylcellulose (low viscosity)
c. 15-40% by weight of hydroxypropyl methylcellulose (high viscosity)
d. 1.5-7.5% by weight of calcium carboxymethyl cellulose
e. 0.1-2% by weight of magnesium stearate.

11. A method for preparing the pharmaceutical formulation according to any of the preceding claims, comprising the steps of
a. mixing together metformin hydrochloride, carboxymethylcellulose sodium, and low viscosity hydroxypropyl methylcellulose,
b. granulating the mixture with a sufficient amount of water,
c. sieving the wet granules formed, drying the same, and sieving the dried granules again,
d. adding high viscosity hydroxypropyl methylcellulose into the resulting mixture and mixing the same,
e. adding magnesium stearate into the final mixture and mixing the same to give a controlled-release phase,
f. mixing together pioglitazone hydrochloride, lactose monohydrate, and calcium carboxymethyl cellulose,
g. adding an aqueous solution of hydroxypropyl cellulose prepared separately into this mixture to provide granules and drying the same,
h. sieving magnesium stearate and mixing it into the resulting mixture to give the immediate-release phase,
i. compressing the controlled-release phase and the immediate-release phase to give a bilayer tablet.

12. A solid pharmaceutical combination formulation according to any of the preceding claims for preventing or treating diabetes mellitus in mammalians and particularly in humans.
